# EUROPEAN PATENT SPECIFICATION

(11) **EP 2 558 068 B1**
(45) Date of publication and mention of the grant of the patent: **30.11.2016**
(21) Application number: 11715601.8
(22) Date of filing: 11.04.2011
(51) Int. Cl.: A61K 9/00, A61K 9/06, A61K 9/107, A61K 47/36, A61K 47/42, A61K 47/44

(54) **ANTI-ABUSE GELLED PHARMACEUTICAL COMPOSITIONS**
MISSBRAUCHSSICHERE GELIERTE PHARMAZEUTISCHE ZUSAMMENSETZUNGEN
COMPOSITIONS PHARMACEUTIQUES GÉLIFIÉES ANTI-ABUS

(30) Priority: 14.04.2010 GB 201006200
(43) Date of publication of application: 20.02.2013
(73) Proprietor: Ayanda Group AS, 0667 Oslo (NO)
(72) Inventor: DRAGET, Kurt, Ingar, 0667 Oslo (NO); HAUG, Ingvild, Johanne, 0667 Oslo (NO); ENGELSEN, Steinar, Johan, 0667 Oslo (NO); SETERNES, Tore, 0667 Oslo (NO); HATTREM, Magnus, N, 0667 Oslo (NO)
(74) Representative: Robinson, Alexander Joseph
(86) International application number: PCT/GB2011/000560
(87) International publication number: WO 2011/128630

(56) References cited:
- WO-A1-99/18967
- WO-A1-2004/054539
- WO-A1-2005/123039
- WO-A2-02/24165
- WO-A2-2006/106344
- WO-A2-2010/041015
- WO-A2-2011/128634

## Description

This invention relates to pharmaceutical compositions in the form of a physiologically tolerable gelled oil-in-water emulsion containing a drug substance of abuse, especially a stimulant, sedative, tranquiliser, strong pain reliever (e.g. an opioid), or a psychoactive agent.

Many drugs which are prescribed for a legitimate use are misused or abused. Three types of drugs are particularly prone to abuse: opioids, CNS depressants, and stimulants. Examples include morphine, morphine-6-glucuronide, diamorphine, hydrocodone, oxycodone, methadone, codeine, diphenoxilate, propoxyphene, dextropropoxyphene, oxymorphone, pentazocine, levorphanol, hydromorphone, buprenorfine, ketobemidone, pethidine, meperidine, oxycodone, fentanyl, tramadol, tapentadol, levorphanol, butorphanol, benzodiazepines (e.g. alprazolam, diazepham), zolpidem, methylphenidate, amphetamines, barbiturates, and pentobarbital.

Such prescription drugs may for example become available for abuse by being stolen from or sold by the legitimate patient. In order to maximise sales or to present the drug in a form suitable for snorting or injection, such drugs are frequently crushed, and optionally diluted and re-tableted or solvent extracted.

A number of strategies have been developed to hinder or prevent such dilution or subsequent abuse. One for example involves including in opioid oral dosage forms an opioid anti-agonist, for example naloxone, which does not block the opioid activity when the oral dosage form is consumed but which will be extracted with the opioid on solvent extraction and will then block the opioid's effect on injection of the extract. A further strategy is to present the drug substance in an inactive pro-drug form, e.g. an enol ester, which requires digestive enzymes to release the active drug. In this case the prodrug is inactive if snorted as a powder or injected following extraction. Other strategies involve incorporating an irritant (e.g. capsaicin) or a bitter component (e.g. denatonium benzoate) to limit snorting or injection abuse.

Still further strategies involve presentation in a hard, not easily crushable dosage form or in a form which gels on addition of water or attempted crushing.

WO 2006/106344 describes hard shell capsules filled with a pharmaceutical medicament and at least one modifier selected to prevent abuse of the medicament.

Such strategies however may risk reducing patient acceptability of the oral dosage form when consumed by the legitimate recipient and there is thus a continuing need for abuse-deterring oral dosage forms for drugs subject to abuse.

We have now found that drugs subject to abuse may be presented in a legitimate user friendly but abuse-deterring form by including the drug substance in a physiologically tolerable gelled oil-in-water emulsion. Since gels are flexible, crushing is rendered difficult and even if crushing is effected under cryogenic conditions, the resulting fragments are not a free flowing powder when returned to ambient temperatures. Solvent extraction is complicated since the extracts will be contaminated by components from the aqueous or oil phase of the gelled emulsion, e.g. lipids or gelling agents. The gelled emulsion however is readily consumed by the legitimate user with no unpleasant effects.

Moreover, if the oil phase in the gelled emulsion contains unsaturated fatty acids (e.g. omega-3, omega-6 or omega-9 fatty acids), for example fish oils, any attempt to crush or to solvent extract the drug of abuse will result in an evil-smelling and tasting product which will be unattractive to abusive users. This arises from the susceptibility of such oils to oxidation.

Thus viewed from one aspect the invention provides an oral pharmaceutical composition according to claim 1.

A colouring agent may be added to the compositions according to the invention in order to further increase the anti-abuse potential thereof. Such colouring agents are preferably lipid soluble, for example canthaxanthin (CAS number 514-78-3) or beta-carotene (CAS number 725-40-7). However, water soluble colouring agents may be used additionally or alternatively, e.g. caramel (CAS number 8028-89-5) or the cochineal extract carmine (CAS number 1260-17-9). Desirably the colour of the colouring agent is unattractive to anyone seeking to inject an extract, e.g. emulsion, stained with it.

If desired, the drug of abuse may be present in delayed or sustained release form. This may be achieved by conventional microencapsulation and dispersion of the encapsulated drug in one or both of the oil and water phases.

By drug of abuse is meant a drug substance or combination of drugs having a legitimate use selected from the group consisting of stimulants, sedatives, tranquilizers, strong pain relievers, and psychoactive agents. By strong pain reliever is meant drugs such as opioids, morphine, codeine, oxycodone, hydrocodone, diamorphine, pethidine, tramadol, buprenorphine, venlafaxine, nefopam, carbamazepine, gabapentin and pregabalin and tricyclic antidepressants such as amitriptyline,but not over-the-counter available analgesics such as acetyl salicylic acid, paracetamol, ibuprofen and other NSAIDs (however some doses and combinations of over the counter drugs may require prescription in certain jurisdictions and such doses/combinations are considered drugs of abuse).

Examples of drugs of abuse include codeine, morphine (and morphine derivatives), hydrocodone, oxycodone, diamorphine, pethidine, tramadol, buprenorphine, propoxyphene, hydromorphone, meperidine, diphenoxylate, barbiturates (e.g. pentobarbital sodium), benzodiazepines (e.g. diazepam, alprazolam and flunitrazepam), amphetamines (e.g. amphetamine, dextroamphetamine, I-lysine-d-amphetamine), methyl phenidate, zolpidem, methadone, mephedrone, tetrahydrocannabinol, ketamine, clonidine, mexiletine, tapentadol, and others mentioned above. Antitussives and decongestants are also subject to abuse and are therefore also included. Also included are prescription drugs which contain components that themselves are available over the counter (e.g. drugs such as NSAIDs, aspirin, paracetamol and ibuprofen are usually available over the counter but may also be included in prescription-only analgesics). That is, drug combinations that are prescription-only, e.g. Vicodin, are considered drugs of abuse regardless of whether they contain some over the counter drugs. Further drugs of abuse are listed for example in WO 2005/123039.

Drugs of abuse contemplated for inclusion in the compositions of the invention are named in claim 1.

If desired, the drug of abuse may be present in the compositions of the invention in prodrug form, e.g. as an ester, which is activated following oral ingestion.

Also if desired, the compositions of the invention may contain an antagonist to the drug substance, i.e. an agent which on injection will block the uptake of the drug of abuse, for example naloxone where the drug of abuse is an opioid. Preferably such antagonists are ones which are inactive following oral administration.

The drug of abuse may be presented in an aqueous phase and/or in an oil phase in the compositions of the invention, for example in dissolved or dispersed form. In general the compositions of the invention will be in dose unit form. The drug of abuse will typically be present in such dose units at 10 to 100%, especially 50 to 100% of the dose in conventional oral compositions such as tablets or capsules. These dosages are well known for these drugs and need not be discussed further here.

Contemplated herein is a method of treatment of a mammalian subject (either human or non-human) by oral administration to said subject of an effective amount of a drug substance which is a drug of abuse, the improvement comprising administering said drug substance in a physiologically tolerable gelled oil-in-water emulsion.

Viewed from a further aspect the invention provides an oral pharmaceutical composition as defined herein for use in medicine.

Viewed from a further aspect the invention provides an oral pharmaceutical composition as defined herein for use in treatment of a condition responsive to said drug of abuse by oral administration.

Viewed from a still further aspect the invention provides the use of a drug of abuse for the manufacture of an oral pharmaceutical composition as defined herein for use by oral administration in the treatment of a condition responsive to said drug of abuse.

Besides the drug of abuse, the compositions of the invention may contain further components such as nutrients, e.g. lipids, (especially triglycerides and phospholipids, typically of plant or marine animal origin), vitamins, minerals, and folic acid, pH modifiers, viscosity modifiers, flavours, aromas, sweeteners, colorants, antioxidants, etc.

It is particularly preferred that the compositions according to the invention contain a citrus flavour (e.g. orange or lemon oil) in order to mask any remaining oil taste on chewing. It is also particularly preferred that the compositions according to the invention contain xylitol, e.g. as 0.5 to 50% wt., preferably 1 to 40% wt., e.g. 15 to 40% wt., in order to mask both taste and mouth feel. These may be in the aqueous phase or the oil phase (e.g. as a water-in-oil-in water emulsion), or both; however inclusion in the aqueous phase will generally be sufficient.

The gelled emulsion compositions of the invention will be in dose unit form, with each dose unit having a weight of 50 to 3000 mg, especially 100 to 1500 mg, particularly 400 to 1000 mg.

The composition of the invention will be uncoated, i.e. not within a capsule or shell-coating. Accordingly, to avoid water loss during storage, the dose units will conveniently be individually packaged, e.g. in foil wrappers or in the blisters of a blister pack.

The dose units of the gelled emulsion may be formed for example by moulding, extrusion or cutting or the like. For adult use, the dose units are preferably in tablet or lozenge form; however for child use they may conveniently be presented in child-friendly form, e.g. geometric shapes such as rods, strips and tubes, or animal, doll, or vehicle shapes, for example the shape of a popular cartoon character.

The oil phase of the oil-in-water emulsion may be any physiologically tolerable lipid, e.g. fatty acid esters such as triglycerides and phospholipids, for example plant or animal oils, especially plant and marine animal oils. Particularly preferably an oil is used which is high in omega-3, omega-6 or omega-9 essential fatty acids, especially omega-3 essential fatty acids, more especially EPA and DHA. In this way the oil phase itself is a highly bioavailable source of nutrient lipids.

Examples of omega-3 acids include α-linolenic acid (ALA), stearidonic acid (SDA), eicosatrienoic acid (ETE), eicosatetraenoic acid (ETA), eicosapentaenoic acid (EPA), docosapentaenoic acid (DPA), docosahexaenoic acid (DHA), tetracosapentaenoic acid and tetracosahexaenoic acid. Examples of omega-6 acids include linoleic acid, gamma-linolenic acid, eicosadienoic acid, dihomo-gamma-linolenic acid (DGLA), arachidonic acid (AA), docosadienoic acid, adrenic acid, docosapentaenoic acid, and calendic acid. Examples of omega-9 acids include oleic acid, eicosenoic acid, mead acid, erucic acid and nervonic acid.

The oil phase of the oil-in-water emulsion may also contain solubilisers in order to increase the solubility of the drug substance in the oil phase. Suitable solubilisers would be known to a person skilled in the art and include Chremophor EL^{™}, castor oil, Tween 80^{™}, Solutol^{™} HS15, Lutrol^{™} and Olestra.

Other than the drug of abuse, the essential fatty acids may form part or the whole of the oil phase in the gelled emulsion, preferably at least 10% wt, more especially at least 50% wt, particularly at least 80% wt. of that phase. They may be used as single compounds or as compound mixtures, e.g. plant or marine oils.

The aqueous phase of the gelled emulsion will contain water and a physiologically tolerable gelling agent, e.g. a hydrocolloid such as gelatin, alginate, carrageenan or a pectin. Such gelling agents and their gel-forming properties are well known. See for example Phillips GO and Williams PA (Eds.) Handbook of hydrocolloids, Woodhead Publishing, Cambridge (2000). The use of gelatin is especially preferred. Besides water and the gelling agent, the aqueous phase of the gelled emulsion may contain other water-soluble components, e.g. vitamins, minerals, pH modifiers, viscosity modifiers, antioxidants, colorants, flavours, water-soluble drug substances, etc. as desired.

The weight ratio of the lipid phase to the aqueous phase in the gelled emulsions is preferably 1:19 to 3:1, especially 35:65 to 1:1, particularly 2:3 to 1:1.

Emulsion formation may be effected by conventional techniques; however emulsification under a non-oxidising gas, e.g. nitrogen, is preferred. Likewise, the components of the emulsion are preferably degassed before emulsification and handling and packaging of the set emulsion is preferably performed under such a gas.

The gelled emulsions of and used according to the invention may be produced as described in WO 2007/085835 and WO 2007/085840 and PCT/GB2009/002404 and PCT/GB2009/002406.

Anyone trying to abuse the compositions according to the invention in the conventional "street medicine" manner of heating with water in order to extract the drug of abuse and passing the extract through a cigarette filter to remove any contaminants will obtain a milky emulsion rather than the required clear liquid. Since it is a rule among abusers not to inject anything other than clear liquid, the emulsions obtained will prevent abuse. The resulting emulsion can be made even more unattractive by incorporating a colouring agent, especially a lipophilic colouring agent, in the composition of the invention as discussed above.

The invention will now be illustrated further with reference to the following nonlimiting Examples. Examples 3 and 5-9 do not form part of the invention.

### Example 1

### Methylphenidate gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Methylphenidate | 10 mg |
| Water | to 1500 mg |

| | |
|---|---|
| * Total no more than 600 mg | |

The oil(s) and methylphenidate is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 2

### Amphetamine gel

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Amphetamine** | 10 mg |
| Water | to 1500 mg |

| | |
|---|---|
| * Total no more than 600 mg ** several different forms of amphetamine are available and are applicable to the invention. | |

The oil with amphetamine is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 3

### Zolpidem gel

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Zolpidem | 10 mg |
| Water | to 1500 mg |

| | |
|---|---|
| * Total no more than 600 mg | |

The oil with zolpidem is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 4

### Methadone gel

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Methadone | 10 mg |
| Water | to 1500 mg |

| | |
|---|---|
| * Total no more than 600 mg | |

The oil with methadone is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 5

### Phenylephrine gel

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Phenylephrine | 10 mg |
| Water | to 1500 mg |

| | |
|---|---|
| * Total no more than 600 mg | |

The oil with phenylephrine is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 6

### Ephedrine/pseudoephedrine gel

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Ephedrine /pseudoephedrine | 25/60 mg |
| Water | to 1500 mg |

| | |
|---|---|
| * Total no more than 600 mg | |

The oil with ephedrine/pseudoephedrine_is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 7

### Phenylpropanolamine gel

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Phenylpropanolamine | 25 mg |
| Water | to 1500 mg |

| | |
|---|---|
| * Total no more than 600 mg | |

The oil with phenylpropanolamine is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 8

### Dextromethorphan gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Dextromethorphan | 15 mg |
| Water | to 1500 mg |

| | |
|---|---|
| * Total no more than 600 mg | |

The oil with dextromethorphan is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 9

### Noscapine gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Noscapine | 25 mg |
| Water | to 1500 mg |

| | |
|---|---|
| * Total no more than 600 mg | |

The oil with noscapine is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 10

### Morphine gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Morphine | 5 mg |
| Water | to 1500 mg |

| | |
|---|---|
| * Total no more than 600 mg | |

The oil with morphine is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 11

### Tramadol gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Tramadol | 50 mg |
| Water | to 1500 mg |

| | |
|---|---|
| * Total no more than 600 mg | |

The oil with tramadol is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 12

### Tapentadol gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Tapentadol | 50 mg |
| Water | to 1500 mg |

| | |
|---|---|
| * Total no more than 600 mg | |

The oil with tapentadol is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 13

### Hydrocodone gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Hydrocodone | 5 mg |
| Water | to 1500 mg |

| | |
|---|---|
| * Total no more than 600 mg | |

The oil with hydrocodone is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

### Example 14

### Codeine gels

| Components | |
|---|---|
| Gelatin | 84 mg |
| Gum arabicum | 55.5 mg |
| Sorbitol | 155 mg |
| Xylitol | 360 mg |
| Citric acid | 9 mg |
| Flavour | 18 mg |
| Colour | 10.5 mg |
| Plant oil* | 0 - 600 mg |
| Fish oil* | 0 - 600 mg |
| Codeine | 30 mg |
| Water | to 1500 mg |

| | |
|---|---|
| * Total no more than 600 mg | |

The oil with codeine is emulsified with the aqueous phase and the emulsion is poured in aliquots of 1.5 g into elongate moulds lined with a metal/plastics laminate blister tray and allowed to set. The blister tray is thermally sealed with a metal/plastics foil cover sheet.

## Claims

1. An oral pharmaceutical composition in uncoated dose unit form comprising a physiologically tolerable, flexible, gelled biphasic oil-in-water emulsion containing a drug of abuse which is selected from the group consisting of codeine, morphine, hydrocodone, oxycodone, diamorphine, pethidine, tramadol, buprenorphine, propoxyphene, dextropropoxyphene, hydromorphone, oxymorphone, pentazocine, levorphanol, butorphanol, ketobemidone, fentanyl, meperidine, diphenoxylate, venlafaxine, nefopam, carbamazepine, gabapentin, pregabalin, tricyclic antidepressants including amitriptyline, pentobarbital sodium, diazepam, alprazolam, flunitrazepam, amphetamine, I-lysine-d-amphetamine, methyl phenidate, methadone, mephedrone, tetrahydrocannabinol, ketamine, clonidine, mexiletine, and tapentadol, wherein said dose unit has a weight of 50 to 3000 mg.

2. A composition according to claim 1 wherein the drug of abuse is in dissolved or dispersed form.

3. A composition according to claim 1 or claim 2, further comprising an unsaturated fatty acid.

4. A composition according to any preceding claim, further comprising an additional component selected from lipids, vitamins, minerals, folic acid, pH modifiers, viscosity modifiers, flavours, aromas, sweeteners, colorants and antioxidants.

5. A composition according to any preceding claim, further comprising a citrus flavour.

6. A composition according to any preceding claim, further comprising xylitol.

7. A oral pharmaceutical composition in uncoated dose unit form according to any one of claims 1 to 6 for use in medicine.

8. A oral pharmaceutical composition in uncoated dose unit form according to any one of claims 1 to 6 for use in treatment of a condition responsive to said drug of abuse by oral administration.

9. The use of a drug of abuse for the manufacture of an oral pharmaceutical composition in uncoated dose unit form according to any one of claims 1 to 6 for use by oral administration in the treatment of a condition responsive to said drug of abuse.

## Patentansprüche

1. Orale pharmazeutische Zusammensetzung in unbeschichteter Dosierungseinheitsform, umfassend eine physiologisch annehmbare, flexible, gelierte zweiphasige Öl-in-Wasser-Emulsion, die einen Missbrauchsarzneistoff enthält, der ausgewählt ist aus der Gruppe, bestehend aus Codein, Morphin, Hydrocodon, Oxycodon, Diamorphin, Pethidin, Tramadol, Buprenorphin, Propoxyphen, Dextropropoxyphen, Hydromorphon, Oxymorphon, Pentazocin, Levorphanol, Butorphanol, Ketobemidon, Fenatnyl, Meperidin, Diphenoxylat, Venlafaxin, Nefopam, Carbamezepin, Gabapentin, Pregabalin, tricyclische Antidepressiva umfassend Amitriptylin, Pentobarbitalnatrium, Diazepam, Alprazolam, Flunitrazempam, Amphetamin, I-Lysin-d-amphetamin, Methylphenidat, Methadon, Mephedron, Tretrahydrocannabinol, Ketamin, Clonidin, Mexiletin, und Trapentadol, wobei die Dosierungseinheit ein Gewicht von 50 bis 3000 mg aufweist.

2. Zusammensetzung nach Anspruch 1, wobei der Missbrauchsarzneistoff in aufgelöster oder dispergierter Form vorliegt.

3. Zusammensetzung nach Anspruch 1 oder Anspruch 2, ferner umfassend eine ungesättigte Fettsäure.

4. Zusammensetzung nach einem der vorstehenden Ansprüche, ferner umfassend eine zusätzliche Komponente, ausgewählt aus Lipiden, Vitaminen, Mineralien, Folsäure, pH-Modifikatoren, Viskositätsmodifikatoren, Geschmacksstoffen, Aromastoffen, Süßungsmitteln, Farbstoffen und Antioxidantien.

5. Zusammensetzung nach einem der vorstehenden Ansprüche, ferner umfassend einen Zitrusgeschmacksstoff.

6. Zusammensetzung nach einem der vorstehenden Ansprüche, ferner umfassend Xylitol.

7. Orale Zusammensetzung in unbeschichteter Dosierungseinheitsform nach einem der Ansprüche 1 bis 6 zur Verwendung in der Medizin.

8. Orale Zusammensetzung in unbeschichteter Dosierungseinheitsform nach einem der Ansprüche 1 bis 6 zur Verwendung bei der Behandlung eines Zustands, der auf den Missbrauchsarzneistoff durch orale Verabreichung anspricht.

9. Verwendung eines Missbrauchsarzneistoffs zur Herstellung einer oralen pharmazeutischen Zusammensetzung in unbeschichteter Dosierungseinheitsform nach einem der Ansprüche 1 bis 6 zur Verwendung durch orale Verabreichung bei der Behandlung eines Zustands, der auf den Missbrauchsarzneistoff anspricht.

## Revendications

1. Composition pharmaceutique orale sous une forme de dose unitaire non enrobée comprenant une émulsion huile dans l'eau biphasique gélifiée, flexible, physiologiquement tolérable contenant une substance toxicomanogène qui est choisie dans le groupe constitué de codéine, morphine, hydrocodone, oxycodone, diamorphine, péthidine, tramadol, buprénorphine, propoxyphène, dextropropoxyphène, hydromorphone, oxymorphone, pentazocine, lévorphanol, butorphanol, kétobémidone, fentanyl, mépéridine, diphénoxylate, venlafaxine, néfopam, carbamazépine, gabapentine, prégabaline, antidépresseurs tricycliques y compris amitriptyline, pentobarbital sodique, diazépam, alprazolam, flunitrazépam, amphétamine, I-lysine-d-amphétamine, méthylphénidate, méthadone, méphédrone, tétrahydrocannabinol, kétamine, clonidine, mexilétine, et tapentadol, dans laquelle ladite dose unitaire a un poids de 50 à 3000 mg.

2. Composition selon la revendication 1, dans laquelle la substance toxicomanogène est sous une forme dissoute ou dispersée.

3. Composition selon la revendication 1 ou la revendication 2, comprenant en outre un acide gras insaturé.

4. Composition selon l'une quelconque des revendications précédentes, comprenant en outre un composant supplémentaire choisi parmi des lipides, des vitamines, des minéraux, l'acide folique, des modificateurs du pH, des modificateurs de la viscosité, des parfums, des arômes, des édulcorants, des colorants et des antioxydants.

5. Composition selon l'une quelconque des revendications précédentes, comprenant en outre un parfum citron.

6. Composition selon l'une quelconque des revendications précédentes, comprenant en outre du xylitol.

7. Composition pharmaceutique orale sous une forme de dose unitaire non enrobée selon l'une quelconque des revendications 1 à 6 pour une utilisation en médecine.

8. Composition pharmaceutique orale sous une forme de dose unitaire non enrobée selon l'une quelconque des revendications 1 à 6 pour une utilisation dans le traitement d'une affection répondant à ladite substance toxicomanogène par administration orale.

9. Utilisation d'une substance toxicomanogène pour la fabrication d'une composition pharmaceutique orale sous une forme de dose unitaire non enrobée selon l'une quelconque des revendications 1 à 6 pour une utilisation par administration orale dans le traitement d'une affection répondant à ladite substance toxicomanogène.
